# EUROPEAN PATENT APPLICATION

(11) **EP 3 056 233 A1**
(43) Date of publication of application: **17.08.2016**
(21) Application number: 16155108.0
(22) Date of filing: 10.02.2016
(51) Int. Cl.: A61M 1/16, B01D 63/02

(54) **APPLIANCE FOR THE OXYGENATION OF BLOOD**

(30) Priority: 12.02.2015 IT UB20150479
(71) Applicant: Reggiani, Massimo, 41036 Medolla (MO) (IT)
(72) Inventor: Reggiani, Massimo, 41036 Medolla (MO) (IT)
(74) Representative: Zoli, Filippo

(57) **Abstract**

The appliance for the oxygenation of blood (1) comprises a device (2) of the type with radial flow for extracorporeal blood oxygenation having at least an inlet fitting (4) and at least an outlet fitting (5) for the blood arranged so as to define a radial path for the blood itself, at least an inlet duct (6) and an outlet duct (7) for a work gas intended to supply oxygen to the blood and/or to remove carbon dioxide from the same, wherein the device (2) comprises transit means for the work gas placed between the inlet duct (6) and the outlet duct (7) of the type of a bundle of hollow fibers having increasing density going radially outwards,
and by the fact that it comprises at least a main line (9) for the supply of the work gas to the inlet duct (6) and pressure variation means for the variation of the work gas.

## Description

The present invention relates to an appliance for the oxygenation of blood. With particular reference to the field of biomedical devices, devices are known for the extracorporeal oxygenation of the blood of a patient.

In particular, in extracorporeal blood circulation effected to bypass the patient's heart and lungs during cardiac arrest and open heart surgical operations, or else to temporarily help the patient's circulation and breathing in clinical conditions of acute cardiac insufficiency and/or breathing insufficiency (ECMO), blood oxygenation devices are used which are suitable for enriching the blood with oxygen while impoverishing of carbon dioxide, replacing, from a functional viewpoint, the patient's lungs.

The blood oxygenation devices of known type, commonly called "oxygenators", are made up of a casing having an inlet fitting for the venous blood and an outlet fitting for the arterial blood, inside which a work gas is conveyed suitable for yielding oxygen to the blood and for receiving carbon dioxide from it.

A particular type of known oxygenator is the "hollow fiber" type, i.e., characterized by a bundle of hollow fibers arranged inside the oxygenator body and made up of a material semi-permeable to the gases but not to liquids.

These hollow fibers are therefore crossed internally by the work gas and are lapped on the outside by the blood coming from the patient.

Generally, the hollow fibers are made of polypropylene and have pores with a size of less than about 1 µm, so as to allow the gas saturation.

The known oxygenators therefore also comprise an inlet channel and an outlet channel for the work gas, where the inflowing gas consists of oxygen, if necessary mixed with air, and the outflowing gas consists of oxygen and carbon dioxide.

These known devices have some drawbacks among which the fact that their efficiency drops rapidly over time.

In detail, the prolonged contact between the proteins of the blood plasma and the hollow fibers causes the blockage of the latter, preventing the exchange between the inflowing and outflowing work gases.

To this must be added the fact that the polar components of the blood, such as e.g. phospholipids determine the formation of a hydrophilic layer which adheres to the pores of the hollow fibers, blocking them.

In this regard, the fact is underlined that, as it is easy to appreciate, these devices quickly wear out, which does not allow their use for a time longer than about six hours, implying substantial additional costs for their replacement. Another type of oxygenator is known from patent document GB 1 595 058 which describes an appliance for the extracorporeal oxygenation of the blood having an oxygenator and a circuit for supplying gas to same which comprises a main line and a bypass line, between which a valve is interposed for directing the flow of gas towards one of the two lines.

More in detail, along the main line a two-way valve is arranged movable with on/off mode from a control device, which is movable between a closed configuration and an open configuration wherein, the flow of work gas towards the oxygenator is prevented and permitted respectively.

The opening and closing of the two-way valve arranged along the main line permits supplying the gas to the oxygenator in pulsed mode, thus creating peaks of oxygen pressure on the diaphragm of the oxygenator, while the gas supply through the bypass line occurs in a continuous and regular way.

The appliance known from GB 1 595 058 has a number of drawbacks, among which the fact that during the above-mentioned closing of the two-way valve the oxygenator is not supplied and, therefore, the blood is not oxygenated. Another drawback is linked to the fast wear of the hollow fibers that make up the oxygenator caused by the pulsed supply of the work gas.

Furthermore, the switch from the open configuration to the closed configuration of the two-way valve interrupts the flow of work gas facilitating the obstruction of the pores of the hollow fibers and therefore decreasing the deterioration times of same.

The main aim of the present invention is to provide an appliance for the oxygenation of blood which maintains over time a level of greater efficiency compared to devices of known type.

Within this aim, one object of the present invention is to reduce the costs due to the need to frequently replace the oxygenator itself.

Another object of the present invention is to provide an appliance for the oxygenation of blood which greatly reduces the phenomena of obstruction of the pores associated with the flow of blood plasma through the hollow fibers. A further object of the present invention is to provide an appliance for the oxygenation of blood which allows to overcome the mentioned drawbacks of the prior art within the ambit of a simple, rational, easy, effective to use as well as affordable solution.

The above mentioned objects are achieved by the present appliance for the oxygenation of blood having the characteristics of claim 1.

Other characteristics and advantages of the present invention will become better evident from the description of a preferred, but not exclusive, embodiment of an appliance for the oxygenation of blood, illustrated by way of an indicative, but non-limiting, example in the accompanying drawings, wherein :
Figure 1 is a schematic representation of the appliance according to the invention in a first embodiment;
Figure 2 is a schematic representation of the appliance according to the invention in a second embodiment;
Figure 3 is a schematic representation of the appliance according to the invention in a third embodiment;
Figure 4 is a view in section of the oxygenation device according to the invention.

With particular reference to the illustrations, reference number 1 globally designates an appliance for the oxygenation of blood.

The appliance 1 comprises a device 2 of the type with radial flow for extracorporeal blood oxygenation.

The device 2 is of the type known to the expert of the field and is suitable for introducing oxygen into the blood coming from the patient prior to its reintroduction into the patient him/herself.

As known, the device 2 comprises a casing 3 that defines an oxygenation chamber inside which the oxygenation occurs of the blood coming from the patient.

The casing 3 has an inlet fitting 4, which receives the blood to be oxygenated coming from the patient (called venous blood), and an outlet fitting 5 of the blood, through which the oxygenated blood (called arterial blood), and which is to be reintroduced into the patient, comes out.

The inlet fitting 4 is connectable to other devices of the appliance 1, not shown in the illustrations, such as a supply pump suitable for keeping the blood in circulation, e.g. a roller pump.

The casing 3 therefore has an inlet duct 6 and an outlet duct 7 for the work gas. More in detail, the inlet duct 6 is crossed by oxygen, possibly mixed with air, and the outlet duct 7 is crossed by oxygen and carbon dioxide.

As can be seen in the illustrations, the blood enters the device 2 through the inlet fitting 4 arranged centrally with respect to the casing 3 and propagates radially towards the outside of the casing itself.

In particular, during the operation of the appliance 1, the work gas is intended to supply oxygen to the blood and/or to remove carbon dioxide from the same. Inside the device 2, and more in particular inside the casing 3, are arranged transit means of the work gas, not shown in the figure.

The transit means of the work gas are of the type known to the expert of the field and are made up of a bundle of hollow fibres semi-permeable to the gas, placed between the inlet duct 6 and the outlet duct 7 and communicating with the same.

The hollow fibres are therefore crossed internally by oxygen and lapped on the outside by the blood.

Preferably, the hollow fibers located inside the casing 3 are wrapped with the "ball" technique, i.e., they have increasing density moving radially outwards. This type of winding, unlike the common fiber weaving technique of the "carpet" type, allows keeping the fibers separate from each other.

In the present case, the bundle of hollow fibers is wrapped in concentric areas which have increasing winding density going from the central portion of the casing 3 towards the peripheral portion of the casing itself.

This way, the winding density increases radially, contextually to the path followed by the blood.

The blood flowing from the center to the outside of the casing 3 meets a growing volume of hollow fibers; this balances the increasing volume which the blood has at disposal as it gradually moves away from the central portion of the oxygenator.

The exchange between oxygen and carbon dioxide therefore occurs in an incremental way along the radial direction of blood flow inside the device 2, reducing the drop in blood pressure and therefore also the pressure applied by the blood itself against the hollow fibers and, accordingly, greatly reducing the phenomena of obstruction of the pores associated with the passage of blood plasma proteins through the hollow fibers themselves.

According to the invention, the appliance 1 comprises at least a main line 9 for the supply of the work gas to the inlet duct 6 and pressure variation means for the variation of the work gas pressure itself.

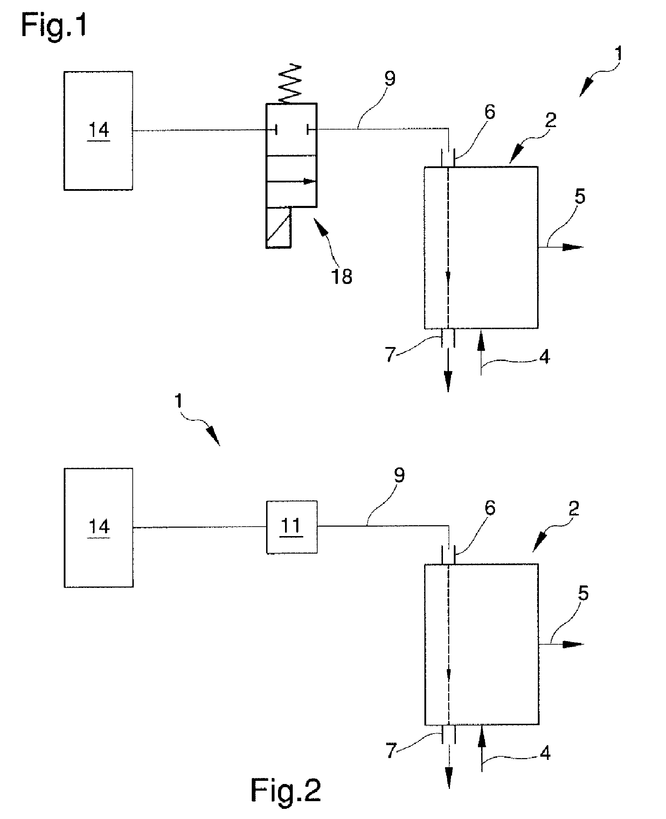

In a first embodiment, shown in figure 1, the pressure variation means are of the type of first valve means 18 operable to open and close the main line 9. The first valve means 18 therefore have two operating configurations, namely an open one, wherein they allow the transit of the gas flow towards the device 2, and a closed one, wherein they block the main line 9 preventing the gas from reaching the device 2. Through the opening and closing of the first valve means 18, pressure peaks therefore occur inside the device 2, which allow, along with the radial flow imposed on the blood by the oxygenation device itself, reducing the obstruction of the pores and increasing the life of the oxygenation device 2.

In the second embodiment according to the invention, shown in figure 2, the pressure variation means are of the type of adjusting means 11 for adjusting the flow rate of the work gas arranged along the main line 9. The adjusting means 11 are e.g. of the type of a two-way valve operable to adjust the gas transit section.

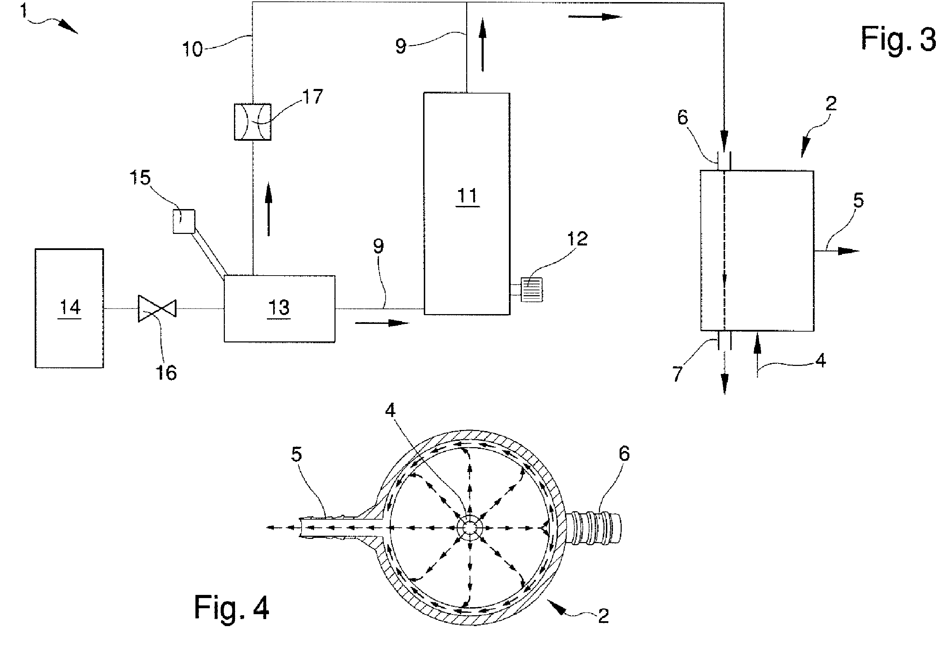

In the third embodiment according to the invention, shown in figure 3, the pressure variation means comprise adjusting means 11 for adjusting the flow rate of the work gas arranged along the main line 9, for the supply of gas at a first pre-settable flow rate, and comprise a bypass line 10 for the supply of the work gas to the inlet duct 6 at a second substantially constant flow rate.

The main line 9 and the bypass line 10 are both suitable for supplying the gas to the device 2 in a continuous way. The flow of gas towards the device 2 is not therefore interrupted depending on whether it is supplied through the main line 9 or the bypass line 10.

With reference to the particular embodiment shown in the illustrations, the main line 9 and the bypass line 10 have at least a section in common associated with the inlet duct.

Usefully, the first flow rate can be set by means of the adjusting means 11 for adjusting the flow rate of the work gas, arranged along the main line 9. Advantageously, the second flow rate is greater than or equal to the maximum value of the first flow rate settable by means of the aforesaid adjusting means 11.

In a preferred embodiment shown in the illustrations, the adjusting means 11 comprise a manual adjuster 12, such as e.g. a crank, by means of which the first flow rate can be set.

Alternative embodiments cannot however be ruled out wherein the adjusting means 11 are automated.

Preferably, the adjusting means 11 comprise a flow rate indicator, so that the latter can be easily seen by an operator during the operation of the appliance 1. The appliance 1 comprises second valve means 13 placed between the main line 9 and the bypass line 10 and operable to allow the transit of the work gas to the inlet duct 6 selectively from the main line 9 or from the bypass line 10. Conveniently, the second valve means 13 comprise a three-way valve.

This valve 13 can be, e.g., of the pneumatic or electronic type and is suitable for causing an increase in work gas pressure due to the increase of the flow rate of the same.

In this regard it is useful to point out that the valve 13 is arranged upstream of the adjusting means 11 with respect to the direction of advancement of the work gas.

It is specified that in the present context by "direction of advancement" is meant the path covered by the work gas coming out of the supply source 14 towards the device 2.

In detail, the valve 13 is connectable to a supply source 14 of the work gas and operable to selectively connect the supply source itself and the main line 9 or the bypass line 10; this means that the valve 13 is movable between a normal operating position wherein it puts the supply source 14 in communication with the main line 9, and a bypass position wherein it puts the supply source itself with the bypass line 10.

As shown in the figure, the valve 13 is connected at input to the supply source 14, and at output to the main line 9 and to the bypass line 10.

An alternative embodiment cannot however be ruled out wherein the valve 13 is arranged downstream of the adjusting means 11, relative to the direction of advancement of the work gas.

In this case, the valve 13 is connected at input to the bypass line 10 and to the main line 9, and at output to the inlet duct 6; this means that the valve 13 is movable between the normal operating position wherein it puts the main line 9 in communication with the inlet duct 6, and a bypass position wherein it puts the bypass line 10 with the inlet duct 6.

According to the invention, the appliance 1 also comprises control means 15 for controlling the valve 13, which can be operated to allow the transit of the work gas towards said inlet duct 6 selectively from said main line 9 or from said bypass line 10.

In particular, the control means 15 are suitable for displacing the valve 13 from the normal operating position to the bypass position.

With reference to the particular embodiment shown in the figures, the control means 15 are operatively connected to the valve 13.

In the present case, the control means 15 comprise a timer suitable for maintaining the valve 13 in the bypass position for a preset time interval. Advantageously, the preset time interval can be changed by the operator depending on the different needs of the operator him/herself, or according to specific clinical contingencies.

Conveniently, the timer can be an electronic, or pneumatic, or hydraulic timer. In a particular embodiment shown in the illustrations, the appliance 1 comprises a pressure reducer 16 placed between the supply source 14 and the valve 13. The pressure reducer 16 is suitable for providing a constant pressure, with respect to the direction of advancement of the work gas downstream of the supply source 14.

In other words, the pressure reducer 16 is suitable for allowing, at inlet of the valve 13, a preset pressure value with respect to the pressure value coming out of the supply source 14, thus avoiding any sudden changes of pressure deriving from the supply source itself.

Preferably, the appliance 1 then also comprises reduction means 17 for reducing the section of the bypass line 10 arranged downstream of the valve 13 and suitable for allowing the transit of the work gas at a second flow rate with a value substantially constant and substantially equal to the maximum value of the first flow rate, which can be set by means of the flow rate adjusting means; this means that the first flow rate is less than or equal to the second flow rate, ensuring a positive, or at most zero increase of the inlet pressure to the device 2. The operation of the present invention is as follows.

The venous blood coming from the patient enters the device 2, through the inlet fitting 4.

At the same time, the valve 13 in normal operating position, allows the transit of the work gas, at a first flow rate preset by the operator using the manual adjuster 12, from the main line 9 to the inlet duct 6.

At the same time, the venous blood inside of the blood compartment 8b laps the hollow fibers externally, yielding carbon dioxide, enriching with oxygen and finally outflowing through the outlet fitting 5.

The timer, once elapsed the time interval previously set by the operator, activates the control means 15 suitable for rotating the valve 13 from the normal operating position to the bypass position.

The work gas flows, therefore, along the bypass line 10 to the inlet duct 6, undergoing a positive pressure increase which releases the hollow fibers from the protein residues of the blood plasma of the device 2.

Once the set time interval has elapsed, the timer activates the control means 15 again suitable for returning the valve 13 to the normal operating position.

More in detail, the valve remains in the bypass position for a shorter time interval with respect to the time it stays in the normal operating configuration.

The method for the oxygenation of blood comprises the following steps of:
- providing an oxygenation device 2 having at least an inlet fitting 4 and at least an outlet fitting 5 for the blood, at least an inlet duct 6 and an outlet duct 7 for a work gas intended to supply the blood with oxygen and/or to remove carbon dioxide from the same;
- first supply of a work gas at a first flow rate;
- second supply of the work gas, at a second flow rate for a preset time interval.

At the end of the second supply, the supply of the work gas is resumed at the first flow rate, so that the second supply defines an increase in the flow rate. Preferably, the steps of first supply and second supply are repeated. Advantageously, the steps of first supply and second supply are carried out repetitively, for a first and a second preset time interval.

More particularly, the first and the second time interval are settable by an operator.

Conveniently, the first time interval is greater than the second time interval.

It has been found in practice how the described invention achieves the intended objects.

In particular the fact is underlined that the appliance according to the invention allows maintaining the effectiveness of the device at optimum levels for a time substantially greater than the devices of known type, cutting costs due to the need to frequently replace the oxygenation device.

To this must be added the fact that the particular expedient of providing an oxygenation device in which blood flows in radial mode and which comprises a winding of the capillary bundle using the "ball" technique, i.e., with a graduated packing density, along with the possibility of setting preset flow rate values permits significantly reducing blood hemolysis thereby positively affecting the deterioration times of the device itself.

This way, the exchange between oxygen and carbon dioxide occurs incrementally along the radial direction of blood flow inside the device, reducing the drop in blood pressure and, therefore, also the pressure applied by the blood itself against the hollow fibers.

In particular, the synergistic combination of the increasing packing density of the hollow fibers proceeding from the central portion towards the marginal portion of the casing and of the positive pressure increase results in the release of the hollow fibers themselves from the protein residues of the blood plasma.

## Claims

1. Appliance for the oxygenation of blood (1), **characterized by** the fact that it comprises a device (2) of the type with radial flow for extracorporeal blood oxygenation having at least an inlet fitting (4) and at least an outlet fitting (5) for the blood arranged so as to define a radial path for the blood itself, at least an inlet duct (6) and an outlet duct (7) for a work gas intended to supply oxygen to the blood and/or to remove carbon dioxide from the same, wherein said device (2) comprises transit means for the work gas placed between said inlet duct (6) and said outlet duct (7) of the type of a bundle of hollow fibers having increasing density going radially outwards,
and by the fact that it comprises at least a main line (9) for the supply of the work gas to said inlet duct (6) and pressure variation means for the variation of the work gas.

2. Appliance (1) according to claim 1, **characterized by** the fact that said pressure variation means are of the type of first valve means (18) operable to open/close said main line (9).

3. Appliance (1) according to claim 1, **characterized by** the fact that said pressure variation means are of the type of adjusting means (11) for adjusting the flow rate of the work gas arranged along said main line (9).

4. Appliance (1) according to claim 1, **characterized by** the fact that said pressure variation means comprise adjusting means (11) for adjusting the flow rate of the work gas arranged along said main line (9) for the supply of gas to said inlet duct (6) at a first pre-settable flow rate and comprise at least a bypass line (10) for the supply of the work gas to said inlet duct (6) at a second substantially constant flow rate, wherein said main line (9) and said bypass line (10) are suitable for supplying the work gas to said device (2) in a continuous way; and by the fact that it comprises second valve means (13) placed between said main line (9) and said bypass line (10) and control means (15) of said second valve means (13) operable to allow the transit of the work gas to said inlet duct (6) selectively from said main line (9) or from said bypass line (10).

5. Appliance (1) according to claim 4, **characterized by** the fact that said second valve means (13) comprise at least a three-way valve.

6. Appliance (1) according to claim 4 or 5, **characterized by** the fact that said second valve means (13) are connectable to a supply source (14) of the work gas and operable to selectively connect the supply source itself and said main line (9) or said bypass line (10), said second valve means (13) being arranged upstream of said adjusting means (11) with respect to the direction of advancement of the work gas.

7. Appliance (1) according to one or more of claims 4 to 6, **characterized by** the fact that said three-way valve (13) can be connected at input to said supply source (14) and connected at output to said main line (9) and to said bypass line (10), said three-way valve (13) being movable between a normal operating position, wherein it puts the supply source (14) in communication with said main line (9), and a bypass position, wherein it puts the supply source itself in communication with the bypass line (10).

8. Appliance (1) according to one or more of claims 4 to 7, **characterized by** the fact that said second flow rate is greater than or equal to the maximum value of said first flow rate settable by means of said adjusting means (11).

9. Appliance (1) according to one or more of claims 5 to 8, **characterized by** the fact that said control means (15) are suitable for displacing said three-way valve (13) from said normal operating position to said bypass position.

10. Appliance (1) according to one or more of claims 5 to 9, **characterized by** the fact that said control means (15) comprise a timer suitable for maintaining said three-way valve (13) in said bypass position for a preset time interval.

11. Appliance (1) according to one or more of claims 4 to 10, **characterized by** the fact that said main line (9) and said bypass line (10) have at least a section in common, associated with said inlet duct (6).

12. Appliance (1) according to one or more of claims 5 to 11, **characterized by** the fact that said three-way valve (13) is of the pneumatic type.

13. Appliance (1) according to one or more of claims 5 to 11, **characterized by** the fact that said three-way valve (13) is of the electronic type.
